# EUROPEAN PATENT SPECIFICATION

(11) **EP 2 767 584 B1**
(45) Date of publication and mention of the grant of the patent: **06.04.2016**
(21) Application number: 13168885.5
(22) Date of filing: 23.05.2013
(51) Int. Cl.: C12M 1/00, B01D 53/84

(54) **Method for reduction of carbon dioxide in biogas**
Verfahren zur Verminderung von Kohlendioxid in Biogas
Procédé de réduction de dioxide de carbone d'un biogaz

(30) Priority: 18.02.2013 EP 13461506
(43) Date of publication of application: 20.08.2014
(73) Proprietor: Politechnika Lódzka, 90-924 Lódz (PL)
(72) Inventor: Zieminski, Krzysztof, 93-355 Lódz (PL); Kopycki, Wlodzimierz, 91-214 Lódz (PL)
(74) Representative: Witek, Rafal

(56) References cited:
- YAOMIN JIN ET AL: "Effects of pH, CO2, and flow pattern on the autotrophic degradation of hydrogen sulfide in a biotrickling filter", BIOTECHNOLOGY AND BIOENGINEERING, vol. 92, no. 4, 20 November 2005 (2005-11-20), pages 462-471, XP55122480, ISSN: 0006-3592, DOI: 10.1002/bit.20607
- MAIKEL FERNÁNDEZ ET AL: "Hydrogen sulphide removal from biogas by an anoxic biotrickling filter packed with Pall rings", CHEMICAL ENGINEERING JOURNAL, vol. 225, 1 June 2013 (2013-06-01), pages 456-463, XP55122164, ISSN: 1385-8947, DOI: 10.1016/j.cej.2013.04.020
- Q. Zhao et al.: "Purification Technologies for Biogas Generated by Anaerobic Digestion", CSANR Research Report, 2010, pages 1-23, XP55122475, Retrieved from the Internet: URL:http://csanr.wsu.edu/wp-content/upload s/2013/02/CSANR2010-001.Ch09.pdf [retrieved on 2014-06-10]
- M Syed ET AL: "Removal of hydrogen sulfide from gas streams using biological processes - A review", Canadian Biosystems Engineering, 2006, pages 2.1-2.14, XP55122483, Retrieved from the Internet: URL:http://engrwww.usask.ca/oldsite/societ ies/csae/protectedpapers/c0436.pdf [retrieved on 2014-06-10]

## Description

The subject of the present invention is a method of reducing the level of carbon dioxide during the desulphuring of biogas conducted in devices for the biological purification of biogas such as biofilters, biofilters with a hydrated layer and bioscrubbers. The present invention belongs to the field of the biotechnological methods of purification of biogas obtained from various sources.

Biogas is a mixture of fermentation gases formed during the activity of anaerobic bacteria that degrade organic substances. This is a material obtained as a result of the decomposition of organic substances by methanogenic anaerobic bacteria, characterised by a variable composition dependent on many factors among which are the initial composition and form of the organic substance, its moisture, pH, the processes temperature and pressure. The biogas mixture usually contains around 52-85% methane (CH₄), 14-48% carbon dioxide (CO₂), 0.08-5.5% hydrogen sulphide (H₂S) as well as low concentrations of nitrogen, oxygen and water vapour. During the production and use of biogas preferred technologies are those that do not harm the environment. As a result of burning low-purity biogas, there may be an increase in the emission of nitrogen and sulphur oxides, as well as carbon oxide and dioxide. Carbon dioxide is seen as one of the main greenhouse gasses that contribute to climate change and environmental devastation. In the future, there will be more restrictive emission indicators in force for the above compounds, which will restrict biogas combustion in gas engines and torches, in some treatment plants and landfills. In order to meet these restrictions, it will be necessary to use solutions ensuring a higher level of purification of biogas than used at present. Key research needs in the area of production and processing of biogas encompass the elimination of the largest possible amounts of undesirable biogas components, in as short a time as possible, with a minimum negative impact on the natural environment, as well as such as the most preferable financial outlay. The presence of carbon dioxide in the biogas has a parallel effect on its calorific value. The calorific value of this fuel is determined by its percentage methane composition. The higher the methane content in the biogas, and thus the lower the content of other components, the higher its calorific value. Carbon dioxide, as a non-flammable gas constitutes a ballast to biogas, greatly reducing its calorific value. Extant technologies for the dry desulphuring of biogas, based on solid absorbents, metal oxides or activated carbon do not remove this ballast, which additionally exacerbates the greenhouse effect. The presence of carbon dioxide in the biogas also cause additional problems with directing the fuel into the natural gas transfer infrastructure, and with its storage by necessitating larger tanks.

Methods of the biological purification of biogas of hydrogen sulphide conducted in devices such as biofilters or bioscrubbers, in which various strains of microorganisms degrading the absorbed contaminants, are known from, amongst others, EP0331806,

WO2008131034 US4760027, WO05037403, US2012026419 US20080245232 US7438886 and Yoamin et al.: Biotech. and Bioeng., 92, 4, 462-471. However, we have not noted information regarding the decisive reduction of the level of carbon dioxide in the biogas in order to resolve the above indicated problems.

There is thus an unresolved need in the state of the art to eliminate the extant obstacles.

The goal of the present invention is to eliminate carbon dioxide from biogas which occurs during the removal of sulphur compounds from the fuel. The purification of biogas occurs using biological methods on devices such as biofilters, bioscrubbers and hydrated biofilters.

Unexpectedly, this goal has been achieved in the present invention.

The basis of the present invention is a method of reducing the level of carbon dioxide during the biological purification of biogas, conducted in devices for the purification of biogas, particularly a biofilter, loaded with a biological filter bed sprinkled with a mineral medium, containing immobilised microorganisms capable of degrading hydrogen sulphide, based on the fact that the process is conducted under anoxic conditions using a consortium of microorganisms collected from natural environments rich in sulphur, the biological filter bed is sprinkled with a medium in the form of a solution containing nitrates of alkali metals of the I and II group, wherein the concentration of nitrate ions in the medium is in the range of 20 to 2500 mg/l, preferably in the range of 50 to 1000 mg/l.

Preferably, the concentration of nitrate ions in the medium is in the range of 50- 200 mg/l.

Preferably, the level of CO₂ in the biogas is reduced by at least 25%.

In a preferable embodiment of the present invention, the consortium of microorganisms contains sulphur bacteria.

Preferably, the process is conducted in biofilters or bioscrubbers.

Preferably, the volume flow intensity of the medium sprinkled onto the biological filter bed is in the range of 5 L/h to 500 L/h per cubic meter of filter bed.

Preferably, the S/N value is from 1 to 30, more preferably 2 to 20.

In a preferable embodiment, the concentration of H₂S during the process is 100 - 3000 ppm, more preferably 300 ppm.

Preferably, the process temperature is 10 to 37°C, preferably 20-27 °C.

Preferably, the pH of the medium is 5-8, preferably no less than 5.5 and no more than 7.8.

A method of reducing the level of carbon dioxide according to the present invention may be embodied in installations for the biological purification of biogas encompassing biofilters, bioscrubbers and biofilters with a hydrated layer.

A method according to the present invention may be conducted in biofilters loaded with filter beds of varying kinds (natural and synthetic). The beds are inoculated with microbes selected from natural conditions rich in sulphur. Microorganisms used in the present invention form a specific group dominated by sulphur bacteria capable of effectively purifying biogas. Research performed in other institutions is based on individual microbial strains. There is a high probability that these microorganisms will not survive industrial conditions, where they will be forced to compete with natural microflora. The effective purification of biogas under industrial conditions may not be successfully conducted by a single strain of bacteria. Providing a consortium of microorganisms from natural conditions rich in sulphur that oxidize hydrogen sulphide is an alternative for difficult industrial conditions.

A medium containing nitrate ions stimulates the decomposition of CO₂ and nitrogen compounds in the biogas, which is very significant to its use as an energy source. The use of nitrates makes it possible to eliminate the stage of aeration of the biogas, as well as greatly easing its purification. In effect, the proposed technology is very competitive to extant solutions.

The process is conducted under anoxic conditions. The purified biogas is not diluted with oxygen, which is significant as this avoids the danger of the formation of an explosive mixture of oxygen and biogas. The lack of a need to aerate also decreases the maintenance costs of the installations. Systems present on the market require aeration. The process according to the present invention does not result in the deposition of elemental sulphur, nor in a decrease of pH, which is a large advantage, since there is no need to remove excess sulphur nor to correct the pH. This is very important, because a common problem in this form of installation is the acidification of the environment. This forces the selection of microorganisms and only those that are tolerant of low pH values develop.

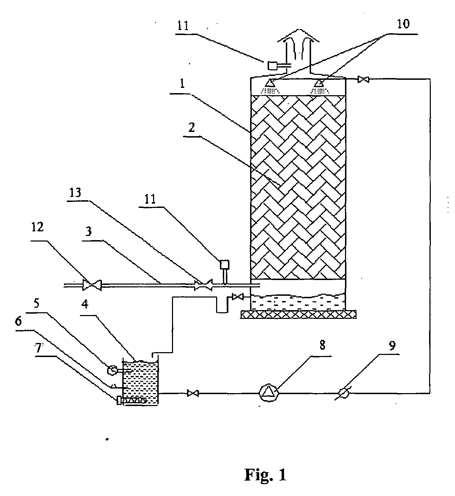

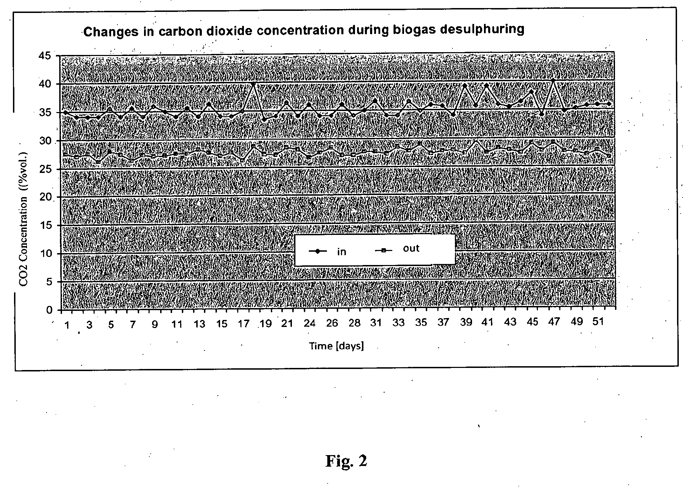

The present invention is defined more closely in the example embodiment shown n the figures, wherein Fig. 1 shows installations for the purification of biogas constructed of biofilters loaded with a carrier. Gaseous contaminants are introduced from the bottom of the device, and recirculated water containing nutrients is sprinkled onto the fill from the top. Microorganisms are immobilised on the carrier. Fig. 2 shows changes in the concentration if carbon dioxide during the desulphuring of biogas.

### Example

An experiment according to the present invention was performed in a sprinkled biofilter acting as a component of an industrial installation. Biogas which formed during methanogenic fermentation is directed under a pressure of 3-5 kPa into a pipeline (3) and thence into the biological desulphuring system. The amount of gas flow is regulated by a flow regulator (13). Biofilter input and output CO₂ concentration measurements and recording are performed using CO₂ concentration sensors (11). The biological filter bed, constituting the filter material (2) with a fill of synthetic material, which makes it possible to form a specific biofilm with a securing activity for the purification of biogas is in the biofilter column (1). To ensure the optimal conditions for the development and selection of microbes, the filter bed of the biofilter is sprinkled from the top (10) with a specific medium containing nitrates. The medium which percolates through the filter beds to the bottom of the column is collected and diverted to an appropriate tank (4). From there, the medium is returned using a pump (8) through a medium flow meter (9) to the sprinkler (10). The medium holding tank (4) is equipped with a heater (7) with a thermostat (5) which makes it possible to maintain a constant temperature, as well as a medium level serisor (6). Biogas flow is cut off with a valve (12).

CO₂ measurements were made at the input and output of the biofilter. The H₂S at the input of the installation was 300 ppm. Fig. 2 shows the difference between the concentration of CO₂ in the biogas supplied into the biofilter system and that diverted from there after the biodegradation of biogas contaminants. The concentration of CO₂ in the biogas supplied into the biofilter was from 33 to 39 % by volume, on average 36 % by vol. Calculated in terms of g/Nm³, this yields 594 g CO₂/ Nm³ to 702 g CO₂/Nm³, the average value was 648 g CO₂/Nm³. The nitrate concentration in the medium was maintained at a level of 200 mg/dm³ to about 50 mg/dm³. Due to the activity of the specific consortium of microorganisms participating in the oxidation of hydrogen sulphide, we obtained an average CO₂ on reduction value of 25%. The concentration of carbon dioxide in the purified biogas was on average 27% by vol. or 486 g CO₂/Nm³. As a result of the desulphuring process of biogas, it is possible to reduce carbon dioxide on average by 162 g/Nm³.

Due to the reduction of the volume of biogas by the amount of carbon dioxide ballast, the present invention makes it possible to effectively utilize biogas as an energy source and also has a beneficial effect on the logistics, as it makes it possible to reduce the size of biogas storage tanks, and facilitates its supply into municipal natural gas infrastructure.

## Claims

1. A method of reducing the level of carbon dioxide during the biological purification of biogas conducted in devices for the purification of biogas, particularly a biofilter loaded with a biological filter bed sprinkled with a mineral medium, containing immobilised microorganisms capable of degrading hydrogen sulphide, **characterised in that** the process is conducted under anoxic conditions using a consortium of microorganisms collected from sulphur-rich natural environments, the biological filter bed is sprinkled with a medium in the form of a solution containing nitrates of alkali metals of the I and II group, wherein the concentration of nitrate ions in the medium is in the range of 20 to 2500 mg/l, preferably in the range of 50 to 1000 mg/l, wherein the level of CO₂ in the biogas is reduced by at least 25%.

2. A method according to Claim 1, **characterised in that** the concentration of nitrate ions in the medium is in the range of 50 to 200 mg/l.

3. A method according to Claim 1, **characterised in that** the consortium of microorganisms contains sulphur bacteria.

4. A method, according to Claim 1, **characterised in that** the purification is conducted in biofilters or bioscrubbers.

5. A method according to Claim 1, **characterised in that** the volume flow intensity of the medium sprinkled onto the biological filter bed is in the range of 5 L/h to 500 L/h per cubic meter of filter bed.

6. A method according to Claim 1, **characterised in that** the S/N value is from 1 to 30, preferably 2 to 20.

7. A method according to any of Claims 1 to 6, **characterised in that** the concentration H₂S during the process is 100 - 3000 ppm, preferably 300 ppm.

8. A method according to any of the above Claims, **characterised in that** the process temperature is 10 to 37°C, preferably 20-27 °C.

9. A method according to any of the above Claims, **characterised in that** the pH of the medium is 5-8, preferably no less than 5.5 and no more than 7.0.

10. A method according to any of the above Claims, **characterised in that** the pH of the medium is 5-8, preferably no less than 5.5 and no more than 7.0.

## Patentansprüche

1. Verfahren zur Reduktion des Kohlendioxidgehalts im Prozess der biologischen Biogasreinigung, der in einer Biogasreinigungseinrichtung durchgeführt wird, insbesondere in einem Biofilter, das mit einem biologischen, mit einem Mineralmedium berieselten Festbett gefüllt ist, welches immobilisierte, den Schwefelwasserstoff zersetzende Mikroorganismen enthält, **dadurch gekennzeichnet, dass** der Prozess unter anaeroben Bedingungen und unter Anwendung eines Konsortiums von aus natürlichen schwefelreichen Ökosystemen stammenden Mikroorganismen durchgeführt wird, und dass das biologische Festbett mit einem Medium in Form von einer Lösung, die Nitrate des Salze der Alkalimetalle der 1. und der 2. Spalte des Periodensystems enthält, berieselt wird, wobei die Konzentration der Nitrat-Ionen im Medium zwischen 20 und 2500 mg/l, insbesondere zwischen 50 und 1000 mg/l liegt.

2. Verfahren nach Anspruch 1, **dadurch gekennzeichnet, dass** die Konzentration der Nitrat-Ionen im Medium zwischen 50 und 200 mg/l liegt.

3. Verfahren nach Anspruch 1, **dadurch gekennzeichnet, dass** der CO₂-Gehalt im Biogas um mindestens 25% reduziert ist.

4. Verfahren nach Anspruch 1, **dadurch gekennzeichnet, dass** das Konsortium von Mikroorganismen Schwefelbakterien enthält.

5. Verfahren nach Anspruch 1, **dadurch gekennzeichnet, dass** die Reinigung in Biofiltern oder Biowäschern durchgeführt wird.

6. Verfahren nach Anspruch 1, **dadurch gekennzeichnet, dass** der Volumenstrom des das biologische Festbett berieselnden Mediums zwischen 5 Uh und 500 L/h pro Kubikmeter Festbett liegt.

7. Verfahren nach Anspruch 1, **dadurch gekennzeichnet, dass**, der S/N-Wert 1 bis 30, insbesondere 2 bis 20 beträgt.

8. Verfahren nach einem der Ansprüche 1 bis 7, **dadurch gekennzeichnet, dass** die H₂S-Konzentration im Prozess 100 - 3000 ppm, insbesondere 300 ppm beträgt.

9. Verfahren nach einem der voranstehenden Ansprüche, **dadurch gekennzeichnet, dass** die Temperatur des Prozesses 10 bis 35°C, insbesondere 20-30 °C beträgt.

10. Verfahren nach einem der voranstehenden Ansprüche, **dadurch gekennzeichnet, dass** der pH-Wert des Mediums 5-8, insbesondere nicht weniger als 5,5 und nicht mehr als 7,0 beträgt.

## Revendications

1. Méthode de réduction du niveau de dioxyde de carbone au processus d'épuration biologique du biogaz menée dans le dispositif d'épuration du biogaz, notamment dans un biofiltre, rempli d'un lit biologique arrosé par un milieu de culture minérale contenant des microorganismes immobilisés aptes à la décomposition de sulfure d'hydrogène, **caractérisée en ce que** le processus est mené sous conditions anaérobes en utilisant le consortium de microorganismes prélevés des milieux naturels riches en sulfure, la couche filtrante est arrosée avec le milieu de culture sous forme de solution contenant des nitrates des sels des métaux alcalins du I^{er} et du II^{e} groupe de la classification périodique, la concentration des ions nitrates dans le milieu de culture étant comprise de 20 à 2500 mg/l, de préférence de 50 à 1000 mg/l.

2. Méthode selon la revendication 1, **caractérisée en ce que** la concentration des ions nitrates dans le milieu de culture est comprise de 50 à 200 mg/l.

3. Méthode selon la revendication 1, **caractérisée en ce que** le niveau de CO₂ dans le biogaz est réduit d'au moins 25%.

4. Méthode selon la revendication 1, **caractérisée en ce que** le consortium de microorganismes contient des bactéries sulfuriques.

5. Méthode, selon la revendication 1, **caractérisée en ce que** l'épuration est menée dans les biofiltres ou bioscrubbers.

6. Méthode selon la revendication 1, **caractérisée en ce que** le débit volu-métrique de milieu de culture arrosant le lit biologique est compris de 5 L/h à 500 Uh par mètre cube de lit.

7. Méthode selon la revendication 1, **caractérisée en ce que** la valeur S/N est de 1 à 30, de préférence de2à20.

8. Méthode selon une des revendications de 1 à 7, **caractérisée en ce que** la concentration de H₂S au processus est de 100 à 3000 ppm, de préférence 300 ppm.

9. Méthode selon une des revendications précédentes **caractérisée en ce que** la température de processus est de 10 à 35°C, de préférence 20-30 °C.

10. Méthode selon une des revendications précédentes, **caractérisée en ce que** le pH milieu de culture est de 5-8, de préférence au-dessus de 5,5 et au-dessous de 7,0.
